(19)

Europäisches Patentamt
European Patent Office
Office européen des brevets

(11) **EP 3 579 754 B1**

(12) **EUROPEAN PATENT SPECIFICATION**

(45) Date of publication and mention
of the grant of the patent:
**05.01.2022 Bulletin 2022/01**

(21) Application number: **18709774.6**

(22) Date of filing: **08.02.2018**

(51) Int Cl.:
*A61B 5/12* *(2006.01)* *H04R 25/00* *(2006.01)*
*A61B 5/00* *(2006.01)*

(86) International application number:
**PCT/IB2018/050779**

(87) International publication number:
**WO 2018/146609 (16.08.2018 Gazette 2018/33)**

(54) **METHOD AND APPARATUS FOR MEASURING OTOACOUSTIC EMISSIONS**

VERFAHREN UND VORRICHTUNG ZUR MESSUNG VON OPTO-AKUSTISCHEN EMISSIONEN

PROCÉDÉ ET APPAREIL DE MESURE D'ÉMISSIONS OTOACOUSTIQUES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **09.02.2017 IT 201700014301**

(43) Date of publication of application:
**18.12.2019 Bulletin 2019/51**

(73) Proprietors:
• **Universita' degli Studi di Roma " Tor Vergata"
00133 Roma (IT)**
• **INAIL - Istituto Nazionale per l'Assicurazione
contro gli Infortuni sul Lavoro
00187 Roma (RM) (IT)**

(72) Inventors:
• **MOLETI, Arturo
I-00077 Monte Compatri (RM) (IT)**
• **SANJUST, Filippo
I-00155 Roma (IT)**
• **SISTO, Renata
I-00077 Monte Compatri (RM) (IT)**

• **CERINI, Luigi
I-00046 Grottaferrata (RM) (IT)**

(74) Representative: **De Ros, Alberto et al
Studio Legale Bird & Bird
Via Borgogna, 8
20122 Milano (IT)**

(56) References cited:
**WO-A1-2013/102867    US-A- 5 792 072
US-A1- 2007 219 458**

• **STANZIAL DOMENICO ET AL: "On the physical
meaning of the power factor in acoustics", THE
JOURNAL OF THE ACOUSTICAL SOCIETY OF
AMERICA, AMERICAN INSTITUTE OF PHYSICS
FOR THE ACOUSTICAL SOCIETY OF AMERICA,
NEW YORK, NY, US, vol. 131, no. 1, 1 January
2012 (2012-01-01), pages 269-280, XP012159808,
ISSN: 0001-4966, DOI: 10.1121/1.3664051
[retrieved on 2012-01-13]**

**EP 3 579 754 B1**

**Description**

The present invention relates to a method and an apparatus for measuring otoacoustic emissions. In more detail, the present invention relates to a method and an apparatus for measuring otoacoustic emissions, known also by the acronym OAE, through the estimation of emissions at the ear drum level in the ear canal starting from measurements taken at the entry of the ear canal, which assures high measurement accuracy, making it independent of the boundary conditions that characterise the measurement environment.

In the present description, the expression "boundary conditions that characterise the measurement environment" means in particular the characteristics of the measurement system, such as the resonance properties of the ear canal, as well as the depth where the measuring instruments are positioned within said ear canal.

Otoacoustic emissions are sound signals generated at the cochlear level and measurable in the ear canal both in the absence and in the presence of an acoustic stimulus which may be administered in the ear canal itself. The former are called spontaneous otoacoustic emissions (SOAE), while the latter are known as evoked otoacoustic emissions (EOAE).

In the clinical and audiological field, evoked otoacoustic emissions have been found to provide important information about the state of health and about any dysfunctions of the cochlea. In this regard, the otoacoustic emissions emitted by the ear drum are taken as references, considering as a first approximation that the middle ear has no influence on their transmission.

Although considerable technological advances have been made in recent years, the measurement of the auditory function through otoacoustic emissions is still affected by intrinsic and extrinsic limitations that reduce its clinical applicability, for example as a diagnostic test of the audiometric threshold level.

Intrinsic limitations are tied to the complexity of cochlear mechanics and of the mechanisms for generating otoacoustic emissions which, for each frequency, involve different components with different physiological meaning. Such components can interfere constructively or destructively with each other according to the related phase.

The most significant extrinsic limitations are tied to the resonating characteristics of the environment where the measurement is carried out, i.e. the ear canal, and to the variability of its geometric parameters, such as length or curvature, as well as of the depth of insertion of the probe in the same ear canal in different experimental sessions.

Today, the intrinsic limitations tied to the complexity of the cochlear mechanisms have been successfully addressed, while the extrinsic limitations tied to the individual variability of the impedance of the ear canal-middle ear system.

These extrinsic limitations are mostly due to the fact that the probes carry out measurements referred to the position where they are placed, i.e. substantially at the entry of the ear canal. In practice, the measurement obtained refers to the end of the resonating cavity that is opposite to the one of interest, represented by the ear drum and, consequently, the measured stimulus differs from the one actually transmitted to the ear drum and the measured level of otoacoustic emissions differs from the otoacoustic emissions emitted at the ear drum level, at least because of the resonance effects tied to the geometric characteristics of the ear canal. The differences introduced by such resonance effects can reach significant levels, even of the order of 10-20 dB.

To offset this effect, a known solution is to measure only the stimulus and response pressure at the entry of the cavity and to apply a correction of the measurement obtained, suitable to derive, for the stimulus, the pressure of the signal transmitted to the middle ear (forward pressure) and, for otoacoustic emissions, the pressure of the otoacoustic emission signal actually emitted by the ear drum - see the article by Charaziak and Shera entitled "Compensating for ear-canal acoustics when measuring otoacoustic emissions" in the "Journal of the Acoustical Society of America" of January 2017.

The relationship that ties the stimulus and response pressure at the entry of the cavity to the pressure of the stimulus and otoacoustic emission actually transmitted and emitted by the ear drum, however, critically depends on the resonating characteristics of the ear canal, enclosed between the ear drum and the measuring probe, and hence it is variable according to the specific boundary conditions of the measurement environment.

To identify the correct correction ratio to be applied in each case, it is necessary to model the generator of the stimulus signal emitted by the probe, according to a prior art technique known as Thévenin modelling, and to periodically repeat a first step of calibrating the Thevenin modelling parameters that characterise the stimulus signal generator and, additionally, a second calibration step carried out after inserting the probe into the ear canal.

In particular, the measurement of the Thévenin parameters of the stimulus signal generator of a probe consists of measuring, with an acoustic pressure sensor (e.g. a microphone) that is a part of the probe itself, the response to a stimulus produced by the generator as a function of frequency, within a certain number of cavity of known impedance. From the analysis of an over dimensioned system of equations (in general, 4-6 tubes are used, to derive two parameters) are obtained the complex quantities relating to the Thévenin pressure and impedance of the generator of the probe. Inserting said probe into the ear canal, from the measured frequency response one obtains the reflectivity of the canal, i.e. the parameter based on which the component of the total pressure that propagates from the source (probe) towards the middle ear is calculated, as a function of the pressure measured by the sensor of the probe.

Thus, this measuring technique, in addition to being found to be particularly laborious and complex, may still not offer a sufficient degree of reliability, because both calibration steps can constitute a source of measurement error.

The article by D. Stanzial et al. by the title "On the physical meaning of the power factor in acoustics" in the "Journal of the Acoustical Society of America" of January 2012, describes a method to characterise acoustically an ear canal on the basis of acoustic pressure and acoustic velocity measurements.

At the same time, the same authors filed an Italian patent application that was later extended as an international patent application (publication number WO 2013/102867 A1) relating to an apparatus for clinical-audiometric investigation based on measurements of acoustic pressure and acoustic velocity; said apparatus was thus conceived to characterise acoustically an ear canal. This international patent application mentions otoacoustic emissions at the end of the description (lines 17-21 of page 5), but does not teach in any way how the described apparatus could be used to measure them, either in terms of formulas to compute pressure and velocity measured by the apparatus, or in terms of "schemes" to be adopted to actually be able to measure otoacoustic emissions, nor does it address the problem of deducting, from the measurements made at the entry of the ear canal, the value of the otoacoustic emissions emitted at the level of the ear drum.

In light of the above, the Applicant has noted the need to devise a method and an apparatus able to measure the otoacoustic emissions emitted at the level of the ear drum in a simple, reliable manner.

Thus, the problem constituting the basis of the present invention is to provide a method and an apparatus for measuring otoacoustic emissions that are able to purify the signal from the effect induced by the resonating characteristics of the system consisting of the ear canal and of the middle ear and variable according to the depth of insertion of the probe, with no need to carry out prior calibrations.

In accordance with a first aspect, the invention relates to a method for measuring otoacoustic emissions characterised in that, considering an acoustic wave generated in an ear canal delimited at a first end by an ear drum and at a second end by a probe (partially positioned in an entry area of the ear canal), where the acoustic wave comprises at least one otoacoustic emissions signal component evoked by a stimulus acoustic signal generated by the probe, said component being generated by the ear drum, the method comprises the steps that consist of:

- measuring by means of the probe simultaneously the acoustic pressure p and the velocity v of the acoustic wave;
- on the basis of the measurements of acoustic pressure p and velocity v thus obtained, calculating the intensity $I_{OAE}$ of the evoked otoacoustic emission component on the basis of the equation:

$$I_{OAE_{EPL}} = \left(\frac{W + I_A}{2}\right)\left(1 - 2R_2 \cdot R_1 \cdot \cos(2kL) + (R_2 \cdot R_1)^2\right)$$

with:

$$W(\omega) = \frac{|p(\omega)|^2}{2\rho c} + \frac{\rho c}{2}|v(\omega)|^2$$

$I_A = <pv>$ equal to the active intensity of the measured acoustic wave; equal to the power density of the measured acoustic wave, where $\rho, c$ are respectively the density of the medium (air) and the velocity of propagation of the acoustic waves in the medium; $R_1 = \sqrt{\frac{W - I_A}{W + I_A}}$ equal to the reflectivity factor at the first end of the canal; $R_2 = \sqrt{\frac{W_S - I_{AS}}{W_S + I_{AS}}}$ equal to the reflectivity factor at the second end of the canal; where W and $I_A$ are understood to be measured with reference to the otoacoustic emission signal, while $W_S$ and $I_{AS}$ are respectively the power density and the active intensity measured with reference to the stimulus acoustic signal, and where L (which represents the length of the acoustic cavity, i.e. the distance between probe and ear drum) can be derived from the time delay of the response to the impulse between successive reflections of the measured acoustic wave,

wherein a processing unit (12) comprising software means (13) is used for controlling generation of acoustic stimulus signals by the probe (11) and for processing measurements obtained by the probe (11),
wherein the software means (13) are adapted to carrying out calculations of said equation.

The Applicant has identified the possibility of using an intensimetric technique, noting that it is able to carry out the measurement in a manner that is intrinsically independent from the measuring point in the canal and does not need Thévenin calibration. Specifically, the Applicant has observed that, carrying out intensimetric measurements, in particular the simultaneous measurement of the pressure and of the velocity of the acoustic wave, it is possible to estimate with precision the otoacoustic emissions signal emitted at the level of the ear drum.

In detail, the Applicant has been able to demonstrate that the measurements of active intensity and of power density, both quantities that depend on the pressure and on the velocity of the measured acoustic wave, make it possible to calculate the intensity emitted by the ear drum and thus attributable to the otoacoustic emissions.

As shown by the calculation developed by the Applicant, these measurements make it possible to attain a measurement of the otoacoustic emissions signal at the ear drum level in the absence of calibration that could introduce a measurement error if they are not carried out with high accuracy.

Therefore, the measuring method according to the invention is far simpler to apply, while offering a higher degree of reliability and robustness.

Similarly, in accordance with a second aspect, the invention relates to an apparatus for measuring otoacoustic emissions comprising:

- a probe to be positioned at least in part in an entry area of the ear canal comprising at least one acoustic signal generating source for generating an acoustic stimulus signal to be sent into the ear canal, an acoustic pressure sensor for measuring the acoustic pressure of an acoustic wave in an ear canal, and an acoustic velocity sensor for measuring the acoustic velocity of an acoustic wave in the ear canal; and

- a processing unit comprising software means for controlling the generation of acoustic stimulus signals by the probe and for processing the measurements obtained by the probe,

wherein the software means are adapted to implement the method for measuring otoacoustic emissions described above on the basis of the simultaneous measurements of pressure and of velocity of an acoustic wave in the ear canal.

Advantageously, the apparatus for measuring otoacoustic emissions according to the invention achieves the technical effects described above in relation to the method.

The present invention, in at least one of the aforesaid aspects, can have at least one of the preferred features that follow; such feature in particular can be mutually combined at will to meet specific application need.

Preferably, on the basis of the measurements of acoustic pressure p and velocity v obtained, the intensity $I_{OAE}$ of the emitted otoacoustic emission component is calculated considering $R_2 = 0$.

In this way, the calculation is particular simplified, inasmuch as it is based on the equation:

$$\frac{W + I_A}{2} = I_{OAE_{EPL}}$$

while providing an excellent degree of measurement precision as shown by the comparative example discussed hereafter.

Preferably, the stimulus acoustic signal is equalised in frequency.

More preferably, the stimulus acoustic signal is equalised in frequency at each measurement of otoacoustic emissions.

More preferably, the stimulus acoustic signal is equalised on the basis of the active intensity of the measured acoustic wave.

The measurement of the otoacoustic emissions requires an equalisation in frequency of the stimulus sent by the acoustic stimulus generator, directed at assuring that the signal emitted by the ear drum to the middle ear has the same reference characteristics. Only in this way is it possible to correctly interpret the spectral pattern of the response as a diagnostic of auditory sensitivity as a function of frequency, comparable and able to be correlated to the spectral pattern of the hearing threshold level measured by tonal audiometry.

The simplest technique used to equalise the stimulus, taking into account only the response of the acoustic stimulus generator positioned at the entry of the cavity, consists of assuring that the level of pressure of the stimulus acoustic signal is equal to all the frequencies and corresponds to the target stimulus. This technique is known by the acronym SPL.

Since the stimulus and the response signal of otoacoustic emissions are respectively released and measured at the entry of the ear canal, closed by the probe itself, and by the ear drum, the resonances of this cavity contribute to determine the amplitude read by the pressure sensor of the probe, both with regard to the stimulus itself, and with regard to otoacoustic emissions.

Consequently, measuring, with the pressure sensor of the probe, the pressure as a function of the frequency of the stimulus and equalising this signal, it is not at all assured that the pressure measured at the entry of the canal coincides with the one actually transmitted to the middle ear. In particular, at the frequency of the quarter wave minimum, there is a minimum pressure at the entry of the canal, measured by the probe, that does not correspond to a minimum at the ear drum.

To introduce a correction that takes into account the resonating effects of the canal, a known technique is to measure preventively the Thévenin parameters of the acoustic stimulus generator in order to correct the measured pressure to derive the component of the total pressure that actually propagates towards the middle ear (forward pressure) and to carry out a frequency equalisation based thereon. This technique is known by the acronym FPL (forward pressure level).

The proof of the effectiveness of this technique is the relative invariance of the level of otoacoustic emissions measured by the depth of insertion of the probe into the canal that demonstrates that the effects of the resonances of the canal are correctly taken into consideration.

However, similarly to what is described above with reference to the measurement step, calibration based on the measurement of the Thévenin parameters is a source of inaccuracies if the parameters are not derived correctly, in addition to being laborious and complex.

The Applicants have identified and demonstrated that, within the limit of small reflectivity, calibrating with respect to the intensity transmitted to the middle ear is equivalent to calibrating with respect to the total pressure component that is propagated towards the middle ear. However, calibration with respect to transmitted intensity has been revealed to be advantageously invariant with respect to the resonating properties of the ear canal and to the position of introduction of the probe, reaching the same outcome as the Thévenin calibration, but without requiring laborious measurements of the impedances of the tubes.

Moreover, calibrating with respect to the transmitted intensity advantageously makes it possible to equalise the transmitted stimulus in different additional clinical applications, such as pure tone audiometry, i.e. the psychoacoustic technique within which the hearing threshold is measured as a function of frequency, on the basis of the patient's subjective response to tonal stimuli (individual frequencies) of known intensity.

Preferably, the stimulus acoustic signal comprises at least two tones with close frequency.

More preferably, the stimulus acoustic signal comprises primary tones in a frequency ratio $f_2/f_1$ of between 1.2 and 1.4

Alternatively, the stimulus acoustic signal is a transient stimulus.

According to an additional alternative, the stimulus acoustic signal is sinusoidal.

Preferably, the measured acoustic wave is separated between the at least one component of otoacoustic emission signal evoked by the stimulus acoustic signal and at least one component of stimulus acoustic signal.

To obtain an excellent measurement of otoacoustic emissions, it is preferable, preliminarily, to reduce acoustic noise in the ear canal; this can be achieved, for example, carrying out the measurement in a sound-proof booth and/or enclosing the ear to be measured in an acoustically insulating cup, for example a cup like the one of a headphone.

Preferably, the acoustic velocity sensor of the measuring apparatus according to the present invention is a sonic anemometer.

Preferably, the acoustic signal generating source of the measuring apparatus according to the present invention comprises a loudspeaker.

Preferably, the acoustic pressure sensor of the measuring apparatus according to the present invention is a microphone.

Preferably, at one end of the probe (to be positioned in an entry area of the ear canal) of the measuring apparatus according to the present invention is located the acoustic pressure sensor, the acoustic velocity sensor, and a first end of a conduit adapted to propagate acoustic stimulus signals.

Preferably, the probe of the measuring apparatus according to the present invention comprises two small tubes adapted to propagate two acoustic stimulus signals to a second end of the conduit.

Preferably, the probe of the measuring apparatus according to the present invention is shaped as a rod.

Preferably, the measuring apparatus according to the present invention comprises a cup able to enclose an ear and to insulate it acoustically from the environment.

Preferably, the cup comprises a holed spherical joint for the insertion of the rod and the subsequent orienting of the rod.

Further features and advantages of the present invention will be more readily apparent from the following detailed description of some preferred embodiments thereof, made with reference to the accompanying drawings.

The different features in the individual configurations may be mutually combined at will according to the previous description, if one were to benefit from the advantages specifically resulting from a particular combination.

In these drawings,

- figure 1 is a block diagram of a preferred embodiment of the method for measuring otoacoustic emissions according to the present invention;

- figure 2 is a chart comparing the measurements carried out (a) with equalisation of the stimulus (FPL) but in the absence of correction of the otoacoustic emission signal; (b) with equalisation of the stimulus and correction of the response signal (EPL) according to the state of the art based on Thévenin modelling; and (c) with equalisation of the stimulus and correction of the response according to the method of the invention (P/V);

- figure 3 is a schematic representation of a preferred embodiment of an apparatus for measuring otoacoustic emissions according to the present invention.

In the following description, for the illustration of the figures identical numerals or reference symbols are used to indicate constructive elements with the same function. Moreover, for clarity of illustration, some references are not repeated in all figures.

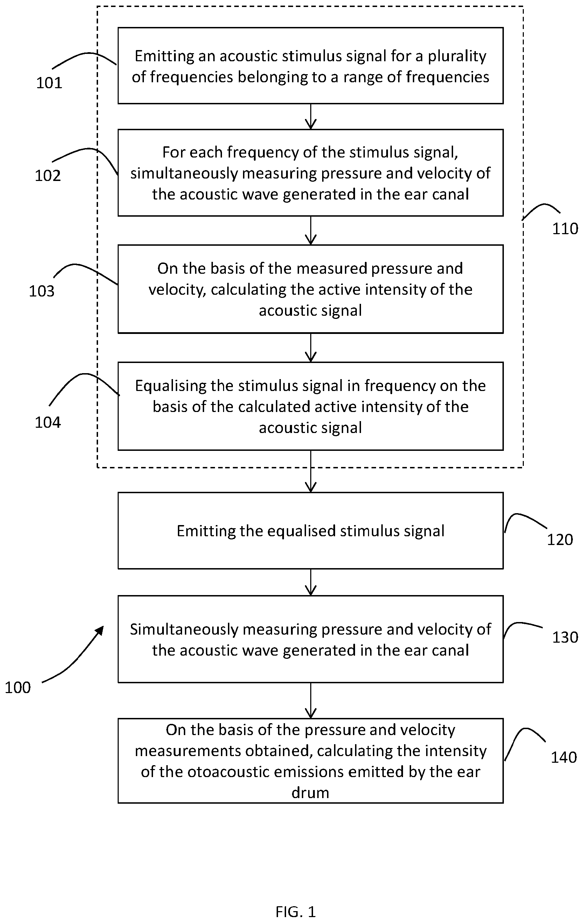

With reference to figure 1, the steps of a method for measuring otoacoustic emissions according to the present invention, indicated in its entirety with the numeral 10, are schematically shown.

Initially, an equalised stimulus signal is generated (step 120).

The stimulus is preferably generated transmitting two tones with close frequency in order to evoke the so-called distortion-product otoacoustic emissions (DPOAE).

This stimulus technique is based on two observations pertaining to the nature of the cochlea, i.e. that the response of the cochlea is non-linear and that the cochlea is tonotopic, i.e. excitation at each frequency is small everywhere except in proximity to its own resonant site.

The non-linearity of the cochlear response implies that if a region is excited simultaneously at two different frequencies $f_1$ and $f_2$ an otoacoustic response is evoked not only at the two primary frequencies, but advantageously also at frequencies that are linear combinations of $f_1$ and $f_2$ (called DPOAE). In particular, the more intense ones (but still weak compared to the stimuli) are observed at the frequency $2f_1$-$f_2$. This technique makes it possible to easily separate in the frequency domain the weak OAE response from the stimuli.

Moreover, the tonotopicity of the cochlea implies that two frequencies can significantly excite the same point of the cochlear membrane only if they are close to each other. For these reasons, the excitation stimulus preferably consists of primary tones in a ratio of frequencies $f_2/f_1$ that is preferably included in the range 1.05 - 1.60, more preferably in the 1.2 - 1.4 range that has been found to be optimal because it is able to generate retrograde waves with relatively high amplitude at the frequency $2f_1$-$f_2$ (included among audible frequencies) which propagate towards the outer year and contribute to the signal that is sensed as DPOAE. For example, the frequency resolution of the Fourier analysis of the sensed signal makes it possible to distinguish otoacoustic emissions from the stimuli.

In a similar manner, it is equally possible to use as stimulation signal a transient or sinusoidal stimulus, measuring respectively the emissions evoked by transient stimuli (TEOAE) or by sinusoidal stimuli (SFOAE); in this latter case, preferably, an additional sinusoidal stimulus with close frequency, called suppressor tone, is also used. In every case in which sinusoidal stimuli are used, it is possible to use chirp stimuli, in which frequency varies slowly over time.

With specific reference to the preventive equalisation of the stimulus signal (step 110) according to the preferred embodiment described, it takes place on the basis of the active signal intensity $I_{AS}$, i.e. the part of energy that abandons the ear canal, propagating remotely.

The Applicants have formally demonstrated that the active intensity $I_{AS}$ of the signal measured by the probe coincides with the intensity $I_T$ actually transmitted by the ear drum 20 to the middle ear. Therefore, equalising with respect to the active intensity $I_{AS}$ is equivalent to equalising the intensity $I_T$ transmitted to the middle ear.

For this purpose, a probe is used that is able to measure simultaneously the pressure p and the velocity v of the acoustic signal. The probe used comprises at least an acoustic signal generating source (for example a loudspeaker), an acoustic pressure sensor (for example a microphone) and an acoustic wave velocity sensor (for example an acoustic anemometer).

An acoustic stimulus signal is emitted for a plurality of frequencies belonging to a range of frequencies (step 101) and subsequently, for each frequency, the pressure p(ω) and the velocity v(ω) of the acoustic wave are measured at a determined frequency co (step 102), and the active intensity $I_{AS}(\omega)$ of the acoustic wave is calculated (step 103) as a real part of the cross spectrum of p(co) and v(ω): $I_{AS}(\omega) = \Re(p(\omega) \cdot \overline{v}(\omega))$

The acoustic signal is thus equalised on the basis of the calculated value of active intensity $I_{AS}$ (step 104). For this purpose, the calculated value of active intensity $I_{AS}$ is compared with a reference value for the equalisation $I_{ref}$. In case of difference between the reference value $I_{ref}$ for the equalisation and the calculated active intensity $I_{AS}$, the acoustic signal generating source is driven in such a way as to offset this difference and to bring the active intensity $I_{AS}$ of the measured signal to the reference level $I_{ref}$.

In this way it is assured that the same signal intensity is always transmitted to the middle ear irrespective of the frequency thereof.

After the step of generating an equalised stimulus signal, the pressure p(ω) and the velocity v(ω) of the acoustic wave are measured (step 130) comprising a component of otoacoustic emission signal evoked by the generated stimulus acoustic signal and a component consisting of the stimulus acoustic signal itself.

In particular, the measured acoustic wave is separated in a known manner between the two signal components (stimulus and otoacoustic emission). The technique for separating the signal components takes place differently as a function of the stimulation technique employed. In the present case in which the stimulation is carried out according to the DPOAE technique, a separation is carried out in the frequency domain.

On the basis of the pressure and velocity measurements obtained, the intensity of the otoacoustic emissions emitted by the ear drum 20 is calculated (step 140) according to the equation:

$$I_{OAE_{EPL}} = \left(\frac{W + I_A}{2}\right)\left(1 - 2R_2 \cdot R_1 \cdot \cos(2kL) + (R_2 \cdot R_1)^2\right)$$

where W is the power density and $I_A$ is the active intensity. It has been seen above that the active intensity $I_A$ depends on the pressure p and on the velocity v of the acoustic wave. Moreover, the power density W can be calculated starting from the pressure p and from the velocity v of the acoustic wave measured according to the following equation:

$$W(\omega) = \frac{|p(\omega)|^2}{2\rho c} + \frac{\rho c}{2}|v(\omega)|^2$$

with $\rho$, c that are respectively the density of the medium (air) and the propagation velocity of the acoustic waves in the medium.

Moreover, the corrective terms that depend on the reflectivity factors $R_1$ and $R_2$ also can be estimated on the basis of the measurements of pressure p and velocity v of the sound wave and in particular on the ratio between active intensity $I_A$ and power density W, using the stimulus signal component in the case of $R_2$, and the component of otoacoustic emission signal itself in the case of $R_1$.

$$R1 = \sqrt{\frac{W - I_A}{W + I_A}}$$

$$R_2 = \sqrt{\frac{W_S - I_{AS}}{W_S + I_{AS}}}$$

In particular, for the estimation of $R_1$ it is advisable to carry out preventively two measurements of the otoacoustic emissions, respectively at low stimulus level and high stimulus level in order to make up for the particularly low signal-noise ratio that characterises the measurements of these signals of otoacoustic emissions.

The measure of L is instead easily obtained from the time delay between subsequent reflections measured with impulsive stimulus, by means of the acoustic pressure sensor or, alternatively, the acoustic wave velocity sensor.

Wanting to facilitate the calculation neglecting the multiple internal reflections subsequent to the first reflection at the probe ($R_2$=0) and considering that the last term of the equation indicated above is generally negligible, the intensity of the otoacoustic emissions $I_{OAE}$ emitted by the ear drum 20 can be obtained on the basis of the following simplified equation:

$$\frac{W + I_A}{2} = I_{OAE_{EPL}}$$

Therefore, starting from the measurements of pressure and velocity of the acoustic signal it is possible to obtain an estimation of the intensity of the otoacoustic emission signal emitted by the ear drum 20. This estimation is invariant with respect to the resonant characteristics of the canal and thus it can be carried out in the absence of prior calibration, therefore being able to operate in a simpler manner and with a lower risk of error introduced as a result of imprecise calibrations.

It is preferable for the step 110 to be carried out every time the steps 120, 130 and 140 are carried out, slightly before the step 110, i.e. that an equalisation immediately precedes a measurement. It can be considered that equalisation lasts for example 1-2 minutes and the measurement lasts for example 2-5 minutes.

Demonstrating the effectiveness of the technique of the invention, the Applicants have compared the estimation of the intensity of the DOPAE response signal obtained with traditional technique and with the method according to the present invention.

In both cases, DPOAE response signals were measured in normally hearing volunteer test subjects, in the frequency range 2f1-2f2 between 1500 and 4500 Hz, with resolution of 20 Hz.

The stimulus levels used were 65 and 55 dB SPL, at the frequencies f1 and f2, with f2=1.22f1. These values are generally considered standard in DPOAE practice. The system was programmed in Labview, using 24-BIT PXI NI-4461 acquisition cards.

COMPARATIVE EXAMPLE:

For the measurement, a high performance traditional probe (Etymotic ER-10X) was used, provided with a Thévenin calibration system that uses a tube of continuously variable length between 30 and 90 mm.

The Thévenin parameters were estimated in a known manner and, on their basis, the reflectivity of the ear canal was estimated to then measure the component of the total pressure that propagates from the source (probe) towards the middle ear, as a function of the pressure measured by the sensor of the probe.

The stimulus signal was then equalised in frequency with reference to the pressure component that propagates towards the middle ear.

The measurement of the pressure $p_{OAEmic}$ of the otoacoustic emission signal taken at the microphone of the probe was lastly employed for the estimation of the intensity of the otoacoustic emission at the ear drum in a known manner according to the following equation:

$$I_{OAE_{EPL}} = \frac{p^2_{OAE_{mic}}}{\rho_0 c} \left( \frac{(1 - 2R_2 \cdot R_1 \cdot \cos(2kL) + (R_2 \cdot R_1)^2}{(1 + R_1)^2} \right)$$

EXAMPLE ACCORDING TO THE INVENTION

For the measurement, a p-v Microflown probe was used, associated with a pair of ER-2 loudspeakers produced by Etymotic Research.

The pressure and the velocity of the acoustic wave were measured simultaneously for each frequency of the range of frequencies indicated above and, on the basis of these measurements, the active intensity $I_A$ of the measured signal was calculated for each frequency. The stimulus signal was then equalised as a function of the calculated active intensity $I_{AS}$.

Lastly, the measurement of the pressure and of the velocity of the otoacoustic emission signal measured at the probe was used for estimating the intensity of the otoacoustic emission at the ear drum 20 in accordance with the invention according to the following equation:

$$\frac{W + I_A}{2} = I_{OAE_{EPL}}$$

where

$$I_A = <pv>$$

and

$$W(\omega) = \frac{|p(\omega)|^2}{2\rho c} + \frac{\rho c}{2} |v(\omega)|^2.$$

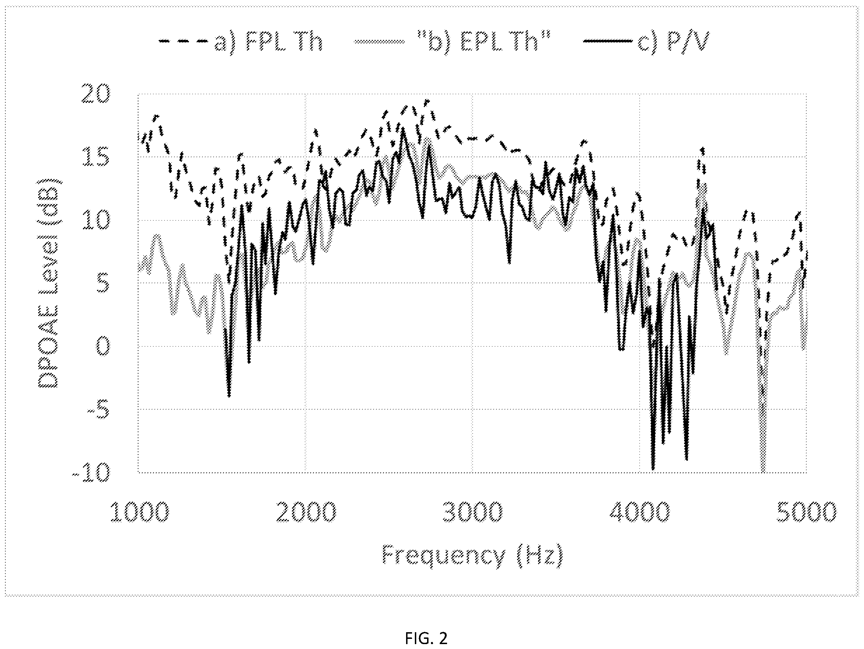

The comparison of the results in the acquisition of otoacoustic distortion products (DPOAE) is shown in figure 2 for an ear of a normally hearing person.

From this chart, it is apparent that the technique according to the invention produces equivalent results to the traditional techniques even with reference to the simplified equation that does not make the correction for infinite internal reflections.

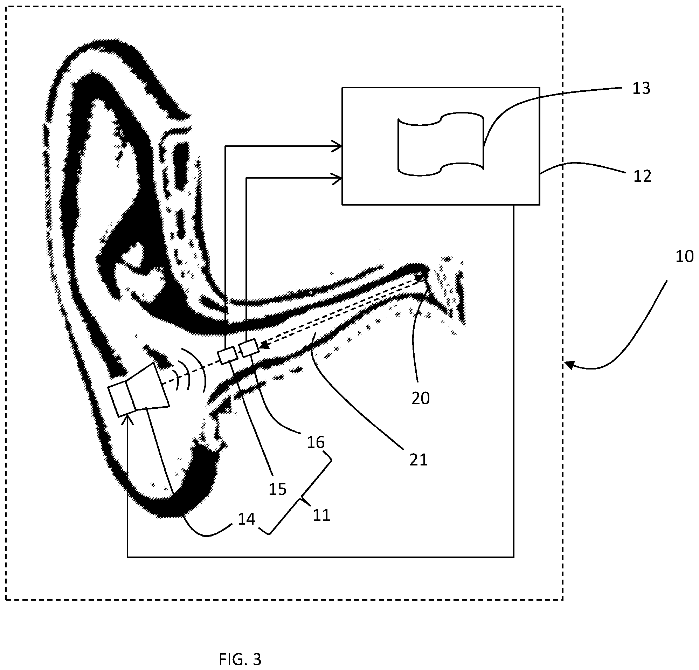

The method for measuring otoacoustic emissions illustrated above is implemented through an apparatus for measuring otoacoustic emissions shown by way of example in Figure 3 and indicated in its entirety with the numeral 10.

The measuring apparatus 10 comprises a probe 11 able simultaneously to measure the acoustic pressure p and the acoustic velocity v of an acoustic wave in an ear canal of an ear, connected to a processing unit 12 comprising software means 13 adapted to implement the measuring method 100 described above, in particular for controlling the generation of acoustic stimulus signals by the probe 11 and for processing measurements taken by the probe 11 (both at the end of the equalisation and at the end of the measurement).

The probe 11 comprises at least one acoustic signal generating source 14, which in the example of figure 3 is a loudspeaker, at least one acoustic pressure sensor 15, which in the example of figure 3 is a microphone, and at least one acoustic wave velocity sensor 16, which in the example of figure 3 is an acoustic anemometer.

Preferably, at one end of the probe 11 (end to be positioned in an entry area of an ear canal) is located the sensor 15, the sensor 16, and a first end of a conduit able to propagate acoustic stimulus signals from the source 14. Moreover, the probe 11 can comprise two small tubes able to propagate two acoustic stimulus signals to a second end of the conduit. As stated, the positioning of the end of the probe 11 is not critical and the probe 11 can be inserted in the ear canal at a depth of 0-10 mm from the entry end of the ear canal.

Preferably, the probe 11 is shaped as a rod.

Preferably, the apparatus 10 also comprises a cup able to enclose an ear and to insulate it acoustically from the environment.

The cup can comprise a holed spherical joint for insertion of the rod and the subsequent orientation of the rod.

From the description provided above, the features of the method and of the apparatus for measuring otoacoustic emissions of the present invention are clear, as are the related advantages.

Additional variants from the embodiments described above are possible, without departing from the scope of the invention.

Lastly, it is clear that method and an apparatus for measuring otoacoustic emissions thus conceived is susceptible to many modifications and variants, all falling within the same inventive concept; furthermore, all details can be replaced by equivalent technical elements. In practice, the materials used, as well as the dimensions, can be of any type according to the technical requirements.

## Claims

1. A method (100) for measuring otoacoustic emissions **characterised in that**, considering an acoustic wave generated in an ear canal (21) delimited at a first end by an ear drum (20) and at a second end by a probe (11) partially positioned in an entry area of the ear canal (21), where the acoustic wave comprises at least one otoacoustic emissions signal component evoked by a stimulus acoustic signal generated by the probe (11), said component being generated by the ear drum (21), said method comprises the steps that consist of:

- measuring (130) by means of the probe (11) simultaneously the acoustic pressure (p) and the velocity (v) of said acoustic wave;
- on the basis of the measurements of acoustic pressure (p) and acoustic velocity (v) obtained, calculating (140) the intensity ($I_{OAE}$) of the evoked otoacoustic emission component on the basis of the equation:

$$I_{OAE_{EPL}} = \left( \frac{W + I_A}{2} \right) \left( 1 - 2R_2 \cdot R_1 \cdot \cos(2kL) + (R_2 \cdot R_1)^2 \right)$$

$$W(\omega) = \frac{|p(\omega)|^2}{2\rho c} + \frac{\rho c}{2} |v(\omega)|^2$$

with: $I_A = <pv>$ equal to the active intensity of the measured acoustic wave; equal

$$R1 = \sqrt{\frac{W - I_A}{W + I_A}}$$

to the power density of the measured acoustic wave; equal to the reflectivity factor at the first

$$R_2 = \sqrt{\frac{W_S - I_{AS}}{W_S + I_{AS}}}$$

end of the canal (20); equal to the reflectivity factor at the second end of the canal (20); where W and $I_A$ are understood to be measured with reference to the otoacoustic emission signal, while $W_S$ and $I_{AS}$ are respectively the power density and the active intensity measured with reference to the stimulus acoustic signal, and where L can be derived from the time delay of the response to the impulse between successive reflections of the measured acoustic wave;

wherein a processing unit (12) comprising software means (13) is used for controlling generation of acoustic stimulus signals by the probe (11) and for processing measurements obtained by the probe (11), wherein the software means (13) are adapted to carrying out calculations of said equation.

2. Method (100) for measuring otoacoustic emissions according claim 1, wherein, on the basis of the measurements of acoustic pressure (p) and acoustic velocity (v) obtained, the intensity ($I_{OAE}$) of the emitted otoacoustic emission component is calculated considering R2 = 0.

3. Method (100) for measuring otoacoustic emissions according to claim 1 or 2, wherein the stimulus acoustic signal is equalised in frequency (110).

4. Method (100) for measuring otoacoustic emissions according to claim 3, wherein the stimulus acoustic signal is equalised in frequency (110) at every measurement of otoacoustic emissions.

5. Method (100) for measuring otoacoustic emissions according to claim 3 or 4, wherein the stimulus acoustic signal is equalised on the basis of the active intensity of the measured acoustic wave (104).

6. Method (100) for measuring otoacoustic emissions according to any of the preceding claims, wherein the stimulus acoustic signal comprises at least two tones with close frequency.

7. Method (100) for measuring otoacoustic emissions according to claim 6, wherein the stimulus acoustic signal comprises primary tones in a frequency ratio f2/f1 of between 1.2 and 1.4.

8. Method (100) for measuring otoacoustic emissions according to any of the claims from 1 to 5, wherein the stimulus acoustic signal is a transient stimulus.

9. Method (100) for measuring otoacoustic emissions according to any of the claims from 1 to 5, wherein the stimulus acoustic signal is sinusoidal.

10. Method (100) for measuring otoacoustic emissions according to any of the preceding claims, wherein the acoustic noise in the ear canal (21) is preliminarily reduced.

11. An apparatus (10) for measuring otoacoustic emissions comprising:

- a probe (11) to be positioned at least in part in an entry area of the ear canal (21) comprising at least one acoustic signal generating source (14) for generating an acoustic stimulus signal to be sent into the ear canal (21), an acoustic pressure sensor (15) for measuring the acoustic pressure of an acoustic wave in the ear canal (21), and an acoustic velocity sensor (16) for measuring the acoustic velocity of an acoustic wave in the ear canal (21); and
- a processing unit (12) comprising software means (13) for controlling the generation of acoustic stimulus signals by the probe (11) and for processing the measurements obtained by the probe (11),

wherein the software means (13) are adapted to implement the method (100) for measuring otoacoustic emissions according to any of the preceding claims from 1 to 9 on the basis of the simultaneous measurements of acoustic pressure (p) and of acoustic velocity (v) of an acoustic wave in the ear canal (21).

12. Apparatus (10) for measuring otoacoustic emissions according to claim 11, wherein the acoustic velocity sensor (16) is an acoustic anemometer.

**13.** Apparatus (10) for measuring otoacoustic emissions according to claim 11 or 12, wherein the acoustic pressure sensor (15) is a microphone.

**14.** Apparatus (10) for measuring otoacoustic emissions according to claim 11 or 12 or 13, wherein at one end of the probe (11) to be positioned in an entry area of the ear canal (21) the acoustic pressure sensor (15), the acoustic velocity sensor (16), and a first end of a conduit adapted to propagate acoustic stimulus signals are located.

**15.** Apparatus (10) for measuring otoacoustic emissions according to claim 14, wherein the probe (11) comprises two small tubes adapted to propagate two acoustic stimulus signals to a second end of the conduit.

**16.** Apparatus (10) for measuring otoacoustic emissions according to any of the claims from 11 to 15, wherein the probe (11) is shaped as a rod.

**17.** Apparatus (10) for measuring otoacoustic emissions according to any of the claims from 11 to 16, comprising a cup able to enclose an ear and to acoustically insulate it from the environment.

**18.** Apparatus (10) for measuring otoacoustic emissions according to claim 16 and 17, wherein the cup comprises a holed spherical joint for the insertion of the rod and the subsequent orientation of the rod.


**Patentansprüche**

**1.** Verfahren (100) zur Messung otoakustischer Emissionen, **dadurch gekennzeichnet, dass** unter Berücksichtigung einer akustischen Welle, die in einem Gehörgang (21) erzeugt wird, der an einem ersten Ende durch ein Trommelfell (20) und an einem zweiten Ende durch eine Sonde (11) begrenzt ist, die teilweise in einem Eingangsbereich des Gehörgangs (21) positioniert ist, wobei die Schallwelle mindestens eine Signalkomponente otoakustischer Emissionen umfasst, die durch ein von der Sonde (11) erzeugtes akustisches Reizsignal hervorgerufen wird, wobei die Komponente durch das Trommelfell (21) erzeugt wird, wobei das Verfahren die folgenden Schritte umfasst:

- gleichzeitige Messung (130) des Schalldrucks (p) und der Geschwindigkeit (v) der genannten Schallwelle mit Hilfe der Sonde (11);
- um auf der Grundlage der erhaltenen Messungen des Schalldrucks (p) und der Schallgeschwindigkeit (v) die Intensität ($I_{OAE}$) der evozierten otoakustischen Emissionskomponente auf der Grundlage der Gleichung zu berechnen (140):

$$I_{OAE_{EPL}} = \left(\frac{W + I_A}{2}\right)\left(1 - 2R_2 \cdot R_1 \cdot \cos(2kL) + (R_2 \cdot R_1)^2\right)$$

$$W(\omega) = \frac{|p(\omega)|^2}{2\rho c} + \frac{\rho c}{2}|v(\omega)|^2$$

mit: $I_A = <pv>$ gleich der aktiven Intensität der gemessenen Schallwelle; $\qquad$ gleich

$$R1 = \sqrt{\frac{W - I_A}{W + I_A}}$$

der Leistungsdichte der gemessenen Schallwelle; $\qquad$ gleich dem Reflexionsfaktor am ersten

$$R_2 = \sqrt{\frac{W_S - I_{AS}}{W_S + I_{AS}}}$$

Ende des Kanals (20); $\qquad$ gleich dem Reflexionsfaktor am zweiten Ende des Kanals (20); wobei W und $I_A$ so zu verstehen sind, dass sie in Bezug auf das otoakustische Emissionssignal gemessen werden, während $W_S$ und $I_{AS}$ die Leistungsdichte bzw. die aktive Intensität sind, die in Bezug auf das akustische Reizsignal gemessen werden, und wobei L aus der Zeitverzögerung der Antwort auf den Impuls zwischen aufeinander folgenden Reflexionen der gemessenen Schallwelle abgeleitet werden kann;

wobei eine Verarbeitungseinheit (12), die Softwaremittel (13) umfasst, zur Steuerung der Erzeugung von akustischen Reizsignalen durch die Sonde (11) und zur Verarbeitung von durch die Sonde (11) erhaltenen Messungen verwendet wird,
wobei die Softwaremittel (13) zur Durchführung von Berechnungen der Gleichung geeignet sind.

2. Verfahren (100) zur Messung otoakustischer Emissionen nach Anspruch 1, wobei auf der Grundlage der erhaltenen Messungen des Schalldrucks (p) und der Schallgeschwindigkeit (v) die Intensität ($I_{OAE}$) der emittierten otoakustischen Emissionskomponente unter Berücksichtigung von R2=0 berechnet wird.

3. Verfahren (100) zur Messung otoakustischer Emissionen nach Anspruch 1 oder 2, wobei das akustische Reizsignal frequenzentzerrt wird (110).

4. Verfahren (100) zur Messung otoakustischer Emissionen nach Anspruch 3, wobei das akustische Reizsignal bei jeder Messung otoakustischer Emissionen in der Frequenz (110) entzerrt wird.

5. Verfahren (100) zur Messung otoakustischer Emissionen nach Anspruch 3 oder 4, wobei das akustische Reizsignal auf der Grundlage der aktiven Intensität der gemessenen Schallwelle (104) entzerrt wird.

6. Verfahren (100) zur Messung otoakustischer Emissionen nach einem der vorhergehenden Ansprüche, wobei das akustische Reizsignal mindestens zwei Töne mit nahe beieinander liegender Frequenz umfasst.

7. Verfahren (100) zur Messung otoakustischer Emissionen nach Anspruch 6, wobei das akustische Reizsignal Primärtöne in einem Frequenzverhältnis f2/f1 zwischen 1,2 und 1,4 umfasst.

8. Verfahren (100) zur Messung otoakustischer Emissionen nach einem der Ansprüche 1 bis 5, wobei das akustische Reizsignal ein transienter Reiz ist.

9. Verfahren (100) zur Messung otoakustischer Emissionen nach einem der Ansprüche 1 bis 5, wobei das akustische Reizsignal sinusförmig ist.

10. Verfahren (100) zur Messung otoakustischer Emissionen nach einem der vorhergehenden Ansprüche, wobei das akustische Geräusch im Gehörgang (21) vorab reduziert wird.

11. Vorrichtung (10) zur Messung otoakustischer Emissionen, umfassend:

- eine Sonde (11), die zumindest teilweise in einem Eintrittsbereich des Gehörgangs (21) zu positionieren ist, mit mindestens einer akustischen Signalerzeugungsquelle (14) zum Erzeugen eines in den Gehörgang (21) zu sendenden akustischen Reizsignals, einem akustischen Drucksensor (15) zum Messen des akustischen Drucks einer Schallwelle im Gehörgang (21) und einem akustischen Geschwindigkeitssensor (16) zum Messen der akustischen Geschwindigkeit einer Schallwelle im Gehörgang (21); und
- eine Verarbeitungseinheit (12), die Softwaremittel (13) zur Steuerung der Erzeugung akustischer Reizsignale durch die Sonde (11) und zur Verarbeitung der von der Sonde (11) erhaltenen Messungen umfasst,

wobei die Softwaremittel (13) geeignet sind, das Verfahren (100) zur Messung otoakustischer Emissionen nach einem der vorhergehenden Ansprüche 1 bis 9 auf der Grundlage der gleichzeitigen Messungen des Schalldrucks (p) und der Schallgeschwindigkeit (v) einer Schallwelle im Gehörgang (21) durchzuführen.

12. Vorrichtung (10) zur Messung otoakustischer Emissionen nach Anspruch 11, wobei der Schallgeschwindigkeitssensor (16) ein akustisches Anemometer ist.

13. Gerät (10) zur Messung otoakustischer Emissionen nach Anspruch 11 oder 12, wobei der Schalldrucksensor (15) ein Mikrofon ist.

14. Vorrichtung (10) zur Messung otoakustischer Emissionen nach Anspruch 11 oder 12 oder 13, wobei an einem Ende der Sonde (11), die in einem Eingangsbereich des Gehörgangs (21) zu positionieren ist, der Schalldrucksensor (15), der Schallgeschwindigkeitssensor (16) und ein erstes Ende einer Leitung, die zur Weiterleitung von akustischen Reizsignalen geeignet ist, angeordnet sind.

**15.** Vorrichtung (10) zur Messung otoakustischer Emissionen nach Anspruch 14, wobei die Sonde (11) zwei kleine Röhren umfasst, die so geeignet sind, zwei akustische Reizsignale an ein zweites Ende der Leitung zu übertragen.

**16.** Vorrichtung (10) zur Messung otoakustischer Emissionen nach einem der Ansprüche 11 bis 15, wobei die Sonde (11) als Stab ausgebildet ist.

**17.** Gerät (10) zur Messung otoakustischer Emissionen nach einem der Ansprüche 11 bis 16, umfassend eine Schale, die ein Ohr umschließen und akustisch von der Umgebung isolieren kann.

**18.** Vorrichtung (10) zur Messung otoakustischer Emissionen nach Anspruch 16 und 17, wobei die Schale eine gelochte kugelförmige Verbindung für das Einsetzen des Stabes und die anschließende Ausrichtung des Stabes aufweist.

**Revendications**

**1.** Procédé (100) pour la mesure d'émissions oto-acoustiques **caractérisé en ce que**, en considérant une onde acoustique générée dans un conduit auditif (21) délimité à une première extrémité par un tympan (20) et à une seconde extrémité par une sonde (11) partiellement positionnée dans une zone d'entrée du conduit auditif (21), où l'onde acoustique comprend au moins une composante de signal d'émissions oto-acoustiques évoquée par un signal acoustique de stimulation généré par la sonde (11), ladite composante étant générée par le tympan (21), ledit procédé comprend les étapes qui consistent à :

- mesurer (130) au moyen de la sonde (11) simultanément la pression acoustique (p) et la vitesse (v) de ladite onde acoustique ;
- sur la base des mesures de pression acoustique (p) et de vitesse acoustique (v) obtenues, calculer (140) l'intensité ($I_{OAE}$) de la composante d'émission oto-acoustique évoquée sur la base de l'équation :

$$I_{OAE_{EPL}} = \left(\frac{W + I_A}{2}\right)\left(1 - 2R_2 \cdot R_1 \cdot \cos(2kL) + (R_2 \cdot R_1)^2\right)$$

$$W(\omega) = \frac{|p(\omega)|^2}{2\rho c} + \frac{\rho c}{2}|v(\omega)|^2$$

avec : $I_A = <pv>$ étant égal à l'intensité active de l'onde acoustique mesurée ;

$$R1 = \sqrt{\frac{W - I_A}{W + I_A}}$$

étant égal à la densité de puissance de l'onde acoustique mesurée ; étant égal au facteur de

$$R_2 = \sqrt{\frac{W_S - I_{AS}}{W_S + I_{AS}}}$$

réflectivité à la première extrémité du conduit (20) ; étant égal au facteur de réflectivité à la seconde extrémité du conduit (20) ; où W et $I_A$ sont compris comme étant mesurés en référence au signal d'émission oto-acoustique, tandis que $W_S$ et $I_{AS}$ sont respectivement la densité de puissance et l'intensité active mesurées en référence au signal acoustique de stimulation, et où L peut être déduit à partir du retard temporel de la réponse à l'impulsion entre des réflexions successives de l'onde acoustique mesurée ;

dans lequel une unité de traitement (12) comprenant des moyens logiciels (13) est utilisée pour commander la génération de signaux de stimulation acoustiques par la sonde (11) et pour traiter des mesures obtenues par la sonde (11),
dans lequel les moyens logiciels (13) sont conçus pour effectuer des calculs de ladite équation.

**2.** Procédé (100) pour la mesure d'émissions oto-acoustiques selon la revendication 1, dans lequel, sur la base des mesures de pression acoustique (p) et de vitesse acoustique (v) obtenues, l'intensité ($I_{OAE}$) de la composante d'émission oto-acoustique émise est calculée en considérant
R2 = 0.

**3.** Procédé (100) pour la mesure d'émissions oto-acoustiques selon la revendication 1 ou 2, dans lequel le signal acoustique de stimulation est égalisé en fréquence (110).

**4.** Procédé (100) pour la mesure d'émissions oto-acoustiques selon la revendication 3, dans lequel le signal acoustique de stimulation est égalisé en fréquence (110) à chaque mesure d'émissions oto-acoustiques.

**5.** Procédé (100) pour la mesure d'émissions oto-acoustiques selon la revendication 3 ou 4, dans lequel le signal acoustique de stimulation est égalisé sur la base de l'intensité active de l'onde acoustique mesurée (104).

**6.** Procédé (100) pour la mesure d'émissions oto-acoustiques selon l'une quelconque des revendications précédentes, dans lequel le signal acoustique de stimulation comprend au moins deux tonalités ayant une fréquence proche.

**7.** Procédé (100) pour la mesure d'émissions oto-acoustiques selon la revendication 6, dans lequel le signal acoustique de stimulation comprend des tonalités primaires dans un rapport de fréquence f2/fl d'entre 1,2 et 1,4.

**8.** Procédé (100) pour la mesure d'émissions oto-acoustiques selon l'une quelconque des revendications 1 à 5, dans lequel le signal acoustique de stimulation est une stimulation transitoire.

**9.** Procédé (100) pour la mesure d'émissions oto-acoustiques selon l'une quelconque des revendications 1 à 5, dans lequel le signal acoustique de stimulation est sinusoïdal.

**10.** Procédé (100) pour la mesure d'émissions oto-acoustiques selon l'une quelconque des revendications précédentes, dans lequel le bruit acoustique dans le conduit auditif (21) est réduit au préalable.

**11.** Appareil (10) pour la mesure d'émissions oto-acoustiques comprenant :

- une sonde (11) destinée à être positionnée au moins en partie dans une zone d'entrée du conduit auditif (21) comprenant au moins une source de génération de signal acoustique (14) pour générer un signal de stimulation acoustique devant être envoyé dans le conduit auditif (21), un capteur de pression acoustique (15) pour mesurer la pression acoustique d'une onde acoustique dans le conduit auditif (21), et un capteur de vitesse acoustique (16) pour mesurer la vitesse acoustique d'une onde acoustique dans le conduit auditif (21) ; et
- une unité de traitement (12) comprenant des moyens logiciels (13) pour commander la génération de signaux de stimulation acoustiques par la sonde (11) et pour traiter les mesures obtenues par la sonde (11),

dans lequel les moyens logiciels (13) sont conçus pour mettre en œuvre le procédé (100) pour la mesure d'émissions oto-acoustiques selon l'une quelconque des revendications 1 à 9 précédentes sur la base des mesures simultanées de pression acoustique (p) et de vitesse acoustique (v) d'une onde acoustique dans le conduit auditif (21).

**12.** Appareil (10) pour la mesure d'émissions oto-acoustiques selon la revendication 11, dans lequel le capteur de vitesse acoustique (16) est un anémomètre acoustique.

**13.** Appareil (10) pour la mesure d'émissions oto-acoustiques selon la revendication 11 ou 12, dans lequel le capteur de pression acoustique (15) est un microphone.

**14.** Appareil (10) pour la mesure d'émissions oto-acoustiques selon la revendication 11 ou 12 ou 13, dans lequel à une extrémité de la sonde (11) devant être positionnée dans une zone d'entrée du conduit auditif (21), le capteur de pression acoustique (15), le capteur de vitesse acoustique (16), et une première extrémité d'un conduit conçu pour propager des signaux de stimulation acoustiques sont situés.

**15.** Appareil (10) pour la mesure d'émissions oto-acoustiques selon la revendication 14, dans lequel la sonde (11) comprend deux petits tubes conçus pour propager deux signaux de stimulation acoustiques vers une seconde extrémité du conduit.

**16.** Appareil (10) pour la mesure d'émissions oto-acoustiques selon l'une quelconque des revendications 11 à 15, dans lequel la sonde (11) se présente sous la forme d'une tige.

**17.** Appareil (10) pour la mesure d'émissions oto-acoustiques selon l'une quelconque des revendications 11 à 16, comprenant une coquille capable d'encercler une oreille et de l'isoler acoustiquement vis-à-vis de l'environnement.

**18.** Appareil (10) pour la mesure d'émissions oto-acoustiques selon les revendications 16 et 17, dans lequel la coupelle coquille un joint sphérique troué pour l'insertion de la tige et l'orientation ultérieure de la tige.

101 ───┐ Emitting an acoustic stimulus signal for a plurality of frequencies belonging to a range of frequencies

102 ───┐ For each frequency of the stimulus signal, simultaneously measuring pressure and velocity of the acoustic wave generated in the ear canal

110

103 ───┐ On the basis of the measured pressure and velocity, calculating the active intensity of the acoustic signal

104 ───┐ Equalising the stimulus signal in frequency on the basis of the calculated active intensity of the acoustic signal

Emitting the equalised stimulus signal ──── 120

Simultaneously measuring pressure and velocity of the acoustic wave generated in the ear canal ──── 130

100

On the basis of the pressure and velocity measurements obtained, calculating the intensity of the otoacoustic emissions emitted by the ear drum ──── 140

FIG. 1

FIG. 2

FIG. 3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013102867 A1